Europäisches Patentamt

European Patent Office

Office européen des brevets

(11) Publication number: **0 121 775**
A1

## EUROPEAN PATENT APPLICATION

(21) Application number: **84102451.6**

(22) Date of filing: **07.03.84**

(51) Int. Cl.³: **C 12 N 15/00,** C 12 N 9/50
**// C12R1/19**

(30) Priority: **09.03.83 JP 38439/83**

(43) Date of publication of application: **17.10.84**
**Bulletin 84/42**

(84) Designated Contracting States: **AT BE CH DE FR GB IT LI LU NL SE**

(71) Applicant: **Beppu, Teruhiko, No. 5-21, 1-chome, Horinouchi Suginami-ku, Tokyo (JP)**

(72) Inventor: **Beppu, Teruhiko, 5-21, Horinouchi-1-chome, Suginami-ku Tokyo (JP)**
Inventor: **Uozumi, Takeshi, 32-8, Takashimadaira-4-chome, Itabashi-ku Tokyo (JP)**
Inventor: **Nishimori, Katsuhiko, 27-5-303, Hakusan-1-chome, Bunkyo-ku Tokyo (JP)**
Inventor: **Shimizu, Norio, Takayamadanchi 10-10 2374-24, Okubocho, Hitachi-shi (JP)**
Inventor: **Kawaguchi, Yoshiyuki, 15-11, Kyuden-1-chome, Setagaya-ku Tokyo (JP)**
Inventor: **Hidaka, Makoto, 16-21, Nishikata-2-chome, Bunkyo-ku Tokyo (JP)**

(74) Representative: **Patentanwälte Henkel, Pfenning, Feiler, Hänzel & Meinig, Möhlstrasse 37, D-8000 München 80 (DE)**

(54) **Novel expression plasmids and their use in the method for expressing prochymosin coding gene in E. coli.**

(57) Improved methods are provided for replication and expression of prochymosin coding gene in E. coli. Novel expression plasmids having prochymosin coding gene operatively attached to the E. coli trp operon have been developed. These plasmids have been inserted into E. coli host cells by transformation, providing new strains to E. coli with high expression capability. The techniques for preparing such expression plasmids and E. coli strains derived therefrom are described. The modified cells, under appropriate cultivation conditions, produces a substantial amount of prochymosin which on activation (viz., in the form of chymosin) displays milk-clotting activity. The genetically engineered new strains of E. coli have been deposited with the Fermentation Research Institute, Ibaragi, Japan under the Budapest Treaty. The methods are thus useful in the massive production of prochymosin by fermentation, and are superior to the known art in the field.

NOVEL EXPRESSION PLASMIDS AND THEIR USE IN THE METHOD FOR

EXPRESSING PROCHYMOSIN CODING GENE IN E. coli

BACKGROUND OF THE INVENTION

This invention relates in general to Genetic Engineering, and more specifically to the production of recombinant DNA containing prochymosim coding gene, and to the utilization of the same to produce microorganisms for the volume production of prochymosin.

The term, "Genetic Engineering" refers to the in vitro technique of forming recombinant DNA containing exogenous genes and a suitable vector, selecting a DNA on the basis of the desired genetic information, and introducing the selected DNA into a suitable host microorganism, whereby the foreign genetic information becomes part of the genetic complement of the host. The genetically engineered microorganism prepared by this technique is capable of expressing the foreign gene in its cells under appropriate conditions of cultivation, and producing a substance (e.g. enzymes, proteins, hormones or the like) for which the gene will code.

The enzymatic protein chymosin is also known as rennin. It is the major proteolytic protein found in the stomach of the pre-ruminant calf, and is responsible for clotting milk. Chymosin has therefore long been used for coagulating milk casein in cheese making. It has 323 amino acids and a molecular weight of 35,652.

It is recognized that two forms of chymosin (A and B) are possible. These two forms differ at amino acid codon 286 (nucleotide 857). The codon GAT in chymosin A would be codon GTT in chymosin B.

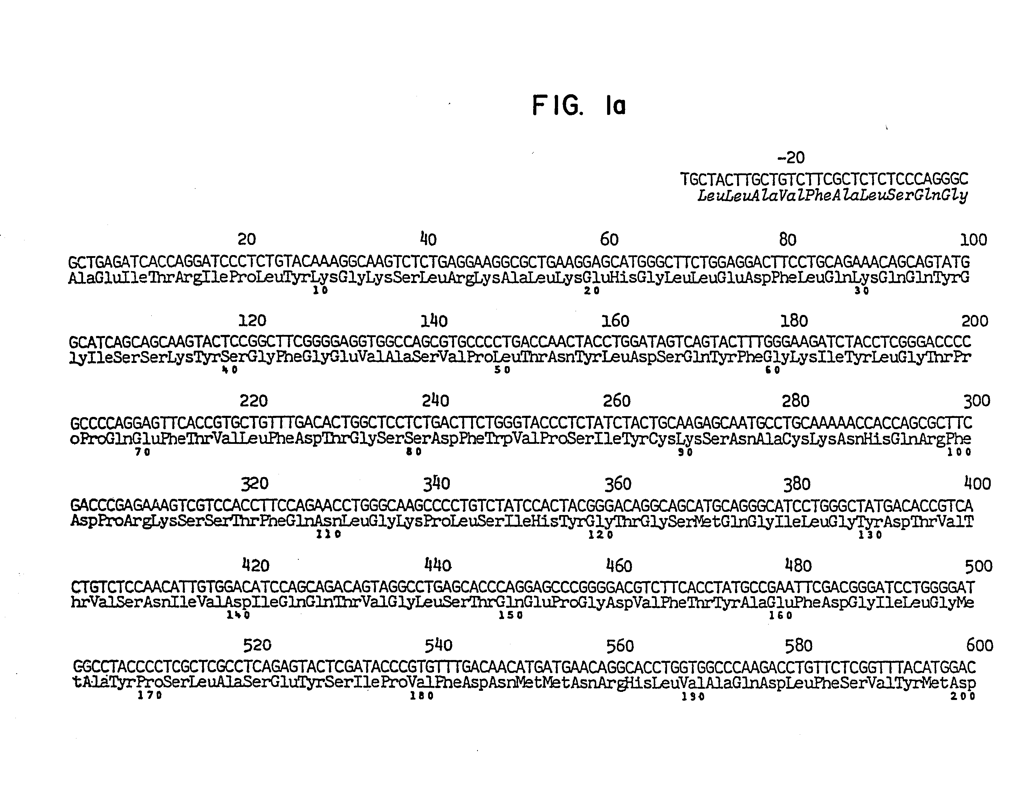

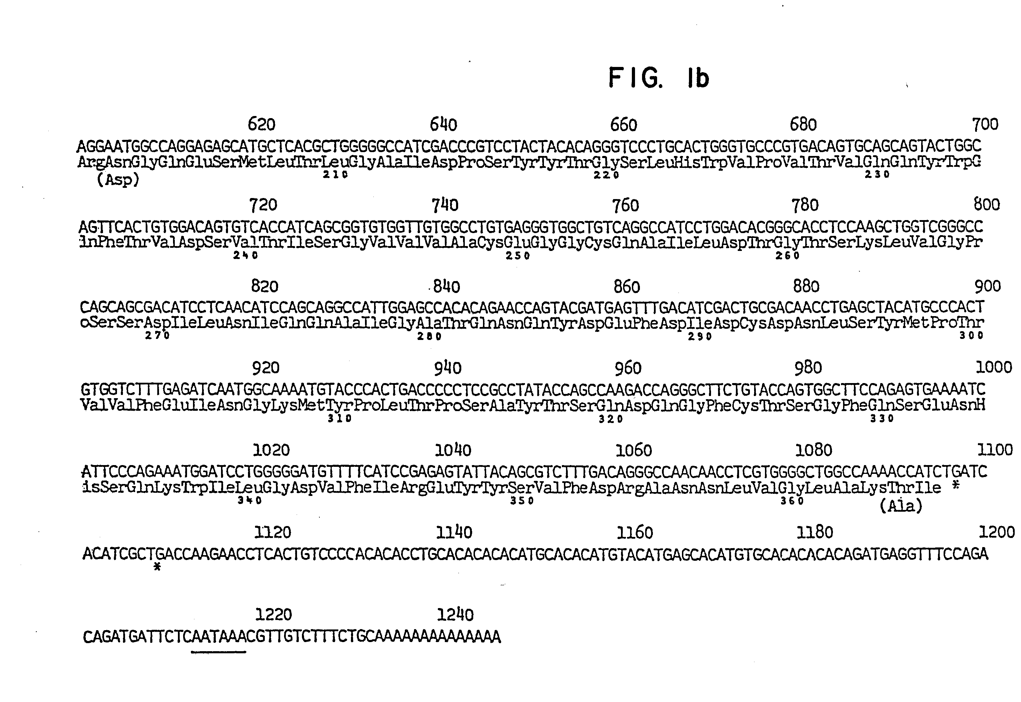

Prochymosin (prorennin) is a precursor of chymosin having 42 additional amino acids at the amino terminus of the chymosin molecule, and it is converted to chymosin under acidic conditions by losing the additional amino acids. Prochymosin has 365 amino acids and a molecular weight of 40,777. The complete amino acid sequence of prorennin has been described in B. Foltmann et al, Proc. Natl. Acad. Sci. USA, 74,231 (1977). The published sequence later proved to be incorrect at a number of amino acid sites. The revised sequence is therefore given in FIG. 1.

Since chymosin is secreted by the mucous membrane of the abomasum of a newly born calf, it is obtained from the stomachs of calves in commercial production. There are several serious problems associated with the obtaining of chymosin from calves: this process involves a number of complicated procedures which present many difficulties when attempting to secure large amounts. It is also costly, and has not been able to meet commercial demands in recent years. For these reasons, some substitutes for chymosin have become acceptable in cheese making. One such substitute is a microbial rennet which is produced by _Mucor_ _pusillous_,

Mucor miehei or Endothia parasitica. However, this microbial rennet has moderately strong proteolytic activity and is not as suitable in cheese ripening as chymosin itself which has a specific yet weak proteolytic activity.

Accordingly, there is still great significance in the supply of a large amount of chymosin from a commercial point of view.

SUMMARY OF THE INVENTION

The process of this invention employs novel expression plasmids which are formed by isolating DNA inserts having prochymosin coding genes from previously constructed recombinant plasmids, and inserting said DNA into suitable vectors. The expression plasmids are then used to transform susceptible microorganisms under conditions where transformation can occur. The microorganism is grown under conditions which will favor the harvesting of transformants which contain the recombinant expression plasmid. Once a host microorganism has been properly transformed, microbial cells can be repeatedly multiplied by culturing the microorganism in an appropriate growth medium, which will express the prochymosin coding gene to produce prochymosin in their cells. Thus, the volume production of prochymosin by microorganisms in fermentation culture will become feasible and provide calf chymosin at reasonable cost.

References relating to this invention are U.S. Pat. No. 4,237,224 (Cohen and Boyer), Japanese Patent Application Sho 56-131,631 to T. Beppu, K. Nishimori, et al, Gene, 19,337 (1982), European Patent Application Publication No. 68691 to Celltech Ltd. and European-Patent Application Publication No. 57350 to Collaborative Research Inc. Particularly, in the copending application Japanese Patent Application Sho 56-131,631 corresponding to Japanese Patent Application Kokai No. 32896/83, filed August 24, 1981 and assigned to the same assignee, an expression plasmid containing the lac UV promoter and almost entire sequence of prochymosin cDNA fued to the N-terminal amino acids of E. coli beta-glactosidase is disclosed.

One of the objects of this invention is to provide specific prochymosin genes for use in the construction of novel expression plasmids.

Another object of this invention is to provide such novel expression plasmids and utilize them to obtain a high expression of prochymosin in microbial host cells.

Another object of this invention is to provide methods for producing novel expression plasmids.

Another object of this invention is to employ the E. coil tryptophan operon promoter in place of the E. coli lac operon promoter.

Still another object of this invention is to obtain genetically engineered microorganisms which will

0121775

be capable of producing substantially greater amounts of prochymosin than could be produced by the hitherto known microorganisms.

A still further object of this invention is to provide a method for producing prochymosin using genetically engineered microorganisms.

One more object of this invention is to provide prochymosin iteself produced according to the above-mentioned methods.

Throughout the Specification, "prochymosin gene" is defined as any sequence of nucleotides which will encode the prochymosin molecule. Any portion of the nuclotide sequence as described in FIG. 1 can be used.

In accordance with a method of this invention, the expression of prochymosin in host cells and their production can be accomplished in terms of the following steps:

1. Specific prochymosin genes are isolated from known recombinant plasmids as described previously by digestion with appropriate restriction enzymes.

2. The prochymosin genes are ligated together with a vector and a translational initiation codon to form an expression plasmid. If the ligated prochymosin genes do not cover the entire coding sequence for prochymosin, an

0121775

additional synthetic oligonucleotide carrying a missing portion of the coding sequence can be fused to the ligated genes.  The synthetic DNA piece may also carry a translational codon preferably preceeding the tip of the prochymosin gene portion.

3.    Suitable host cells are transformed with the expression plasmid by calcium chloride treatment.

4.    Ampicillin-resistant transformed cells are selected.

5.    The transformed cells are grown by standard culturing techniques.

6.    Crude extracts of the cells are tested for the presence of prochymosin by the radio-immunoassay or protein blotting method.

One recombinant plasmid of particular interest is referred to as pCR301, and is described in Nishimori et al., Gene, 19, 337 (1982).  Descriptions of methods for constructing other plasmids carrying prochymosin cDNA may be found in Nishimori et al., J. Biochem., 90, 901 (1981) and other references previously referred to. These plasmids are useful as a source of the prochymosin genes and normally are constructed in the manner described below;

1.    Messenger RNA(mRNA) of the prochymosin gene is isolated from the fourth calf stomach.

2. The mRNA is converted to double-stranded DNA by conventional means.

3. Synthetic linkers are attached to both ends of the double-stranded cDNA.

4. The cDNA molecule is integrated into a suitable vector plasmid (e.g. pBR 322).

5. The recombinant plasmid is then introduced into host cells by transformation.

6. Recognition of correct clones is accomplished by the method of colony hybridization and hybrid-arrested translation.

7. DNA inserts in the clones are sequenced by the method of Maxam and Gilbert.

Protein resulting from a prochymosin coding gene devoid of some tip portion of the coding sequence may be called "prochymosin like protein." Prochymosin having methionine attached to its N-terminus may be called N-terminal methionyl prochymosin. Throughout the Specification, both may be referred to as prochymosin.

As will be later described in detail, by reterring to preferred embodiments, it should be understood that this invention provides:

1. A method for expressing a prochymosin coding gene in E. coli host cells, wherein said gene is derived from transformants containing cloned prochymosin cDNA, said method comprising:

isolating said gene from the transformants; attaching by ligation a transcriptional promoter and a translational initiation codon thereto to form an expression plasmid; and selecting transformed cells that have high levels of expression of prochymosin, N-terminal methionyl prochymosin or prochymosin like protein, said gene having at least from the fifth coding codon to the end of the coding sequence for prochymosin in addition to being fused to the N-terminal portion of the trp L or trp E gene.

2. A method for expressing a prochymosin coding gene in E. coli host cells, wherein said gene is derived from transformants containing cloned prochymosin cDNA, said method comprising:

isolating said gene from transformants; ligating onto said gene a synthetic oligonucleotide carrying a complementary portion of the prochymosin coding sequence which has been deleted from said gene; attaching thereto by ligation a transcriptional promoter and a translatinal initiation codon when not present in said synthetic oligonucleotide to form an expression plasmid; transforming host cells with the expression plasmid; and selecting transformed cells that have high levels of expression of prochymosin or N-terminal methionyl prochymosin, said synthetic oligonucleotide optionally carrying a translational initiation codon.

3. A method for producing prochymosin by means of expression of a prochymosin coding gene in E. coli host cells, wherein said gene is derived from transformants containing cloned prochymosin cDNA, said method comprising; isolating said gene from the transformants; attaching by ligation a transcriptional promoter and a tranlational initiation codon thereto to form an expression plasmid; transforming host cells with the expression plasmid; selecting transformed cells that have high levels of expression of prochymosin, and cultivating the transformed cells in an appropriate nutrient medium.

4. An expression plasmid comprising a prochymosin coding gene and a vector operatively attached thereto, said vector being derived from pBR32 plasmid and containing a transcriptional promoter and a translational initiation codon.

It is also to be understood that this invention encompasses specific prorennin coding gene, coding sequences containing them, methods for constructing novel expression plasmids; transformed microorganisms; and prochymosin produced therefrom.

## BRIEF DESCRIPTION OF THE DRAWING

FIG. 1 shows the complete nucleotide sequence for prochymosin coding gene.

FIG. 2 shows restriction enzyme maps for vector plasmids used in this invention.

FIG. 3 shows restriction enzyme maps for expression plasmids used in this invention.

FIG. 4 shows the DNA sequence in the vicinity of the trp promoter in pOCT2 plasmid.

FIG. 5 is a schematic diagram for the construction of pTRE1 from pOCT3 by way of pOCT2.

FIG. 6 is a schematic diagram for the construction of pTRL1 from pOCT2 and pBR322.

FIG. 7 is a schematic diagram for the construction of pTRP1 from pTRE1.

FIG. 8 is a schematic diagram for the construction of pCR501 from pCR301 and pTRE1.

FIG. 9 is a schematic diagram for the construction of pCR601 from pCR301, pTRE1 and pTRL1.

FIG. 10 is a schematic diagram for the construction of pCR701 from pCR501 and pTRP1.

## DESCRIPTION OF THE PREFERRED EMBODIMENT

## Host Microorganism

Any microorganism capable of accepting and replicating the desired vector plasmids can be used but, for practical reasons, derivatives of <u>E</u>. <u>coli</u> c 600 are used in this invention.  Particularly preferred strain is <u>E</u>. <u>coli</u> c600 $r_k^- m_k^-$  Cells are grown under conventional culturing conditions.  For example, culture media for the <u>E</u>. <u>coli</u> strain can be:

| L Broth | <u>per liter</u> |
|---|---|
| Bacto Tryptone | 10g |
| (Difco Lab., Detroit) | |
| Bacto Yeast Extracts | 5g |
| (Difco Lab., Detroit) | |
| NaCl | 10g |
| Glucose | 2g |

| M9 Broth | <u>per liter</u> |
|---|---|
| $Na_2HPO_4$ | 6g |
| $KH_2PO_4$ | 3g |
| NaCl | 5g |
| $NH_4Cl$ | 1g |
| $CaCl_2$ | 15mg |
| $MgSO_4 \cdot 7H_2O$ | 0.1g |
| B1 (thiamine HCl) | 5mg if required |
| Casamino acid | 2.5g |
| Glucose | 5g |
| Ampicillin | 50mg |
| 3-beta-Indolylacrylic acid | 15mg |

Conventionally, transformation experiments are

- 12 -

0121775

conducted in LB medium containing 50 ug/ml of ampicillin according to the method of Norgard (Gene, 3, 279 (1978). In standard techniques, E. coli cells are grown to a density of from 10 to $30 \times 10^{12}$ cells per liter at a temperature of from 30°C to 40°C.

## Promoter

In order to have cloned genes expressed in host cells, it is necessary to equip vector plasmids with a suitable promoter; several promoters can be used to obtain expression as long as they are compatible with the host cells. Desirably, the E. coli tryptophan operon promoter is used in this invention.

## Recombinant Plasmids

### pCR301

In accordance with this invention, genes are conveniently available from the primary recombinant plasmids as described previously. These plasmids contain a prorennin coding gene with variable sizes and some of them have been mapped precisely by restriction enzyme digestion. Therefore, by cutting with appropriate restriction enzymes, useful portions of the prorennin coding genes can be separated, and used as a source of DNA for a second cloning into a vector plasmid. A representative of such plasmids is pCR301. Plasmid pCR301 contains the lactose operon promoter and almost entire prochymosin gene sequence (from nucleotide #13 to #1095) only devoid of the first four codons.

This plasmid is derived from pBR322 plasmid (F. Bolvar et al., Gene, 2, 95 (1977). The necleotide sequence between the initiation codon ATG and the beginning of the cloned procymosin gene is illustrated below(this sequence coresponds to the nucleotide coding for the ten N-terminal amino acids of beta-glactosidase):

$$5' - \underline{ATG} \text{ GCC ATG TTA ACG GAT TCA CTG}$$
Met

beta-glactosidase

$$\text{GAA TTC } \underline{\text{CGG}} - 3'$$
Arg

prochymosin

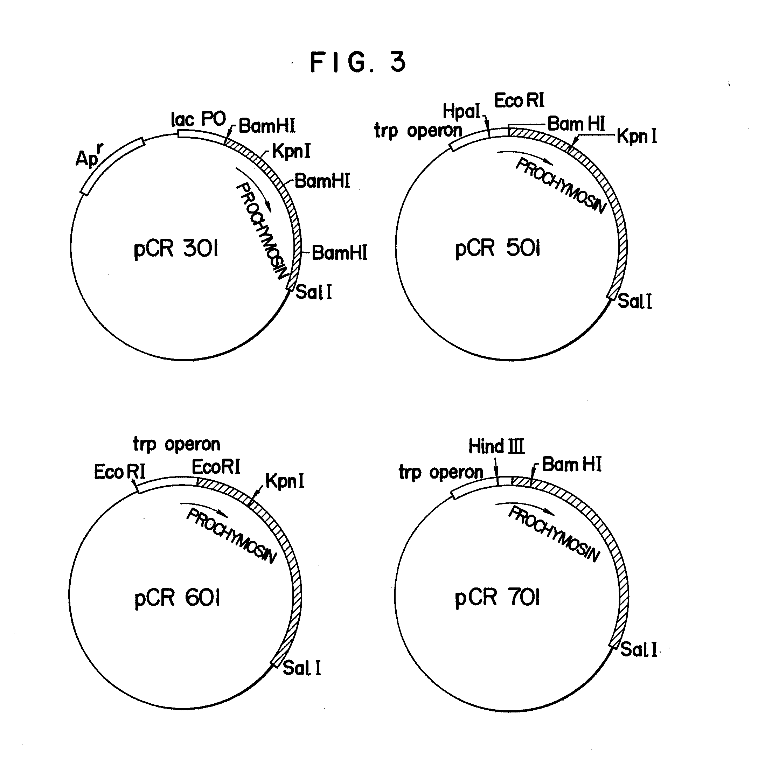

A restriction enzyme map for pCR301 is shown in FIG. 3.

pOCT2

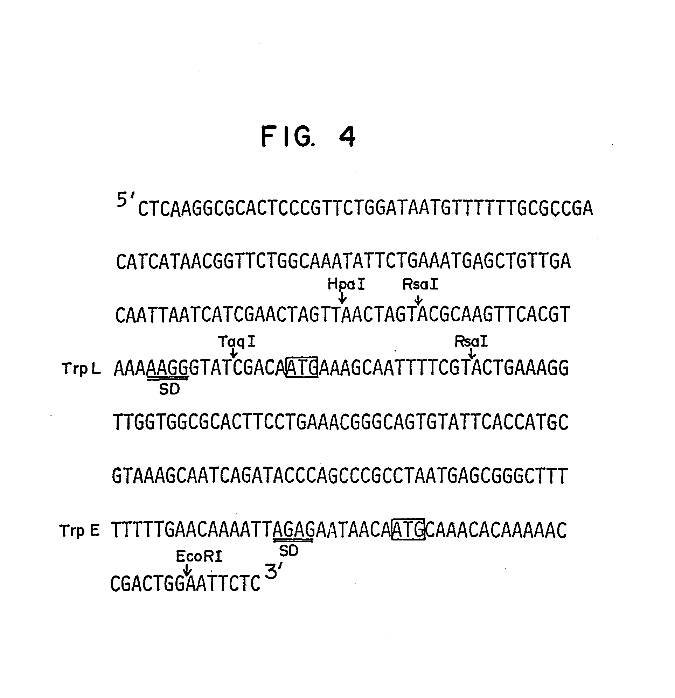

Plasmid pOCT3, the precursor of pOCT2, is a pBR322 plasmid into which a DNA fragment having the trp promoter-operator, trp L and trp E' has been integrated at its EcoRI site. Said trp promoter-operator portion (- 0.5 Kb) is originally extracted from plasmid p trp ED5-1 (R.A. Hallewell and S. Emtage, Gene, 9, 27 (1980)). This DNA is filled in by means of $T_4$ D polymerase, to which a EcoRI linker is annexed. The resulting DNA fragment is inserted into the EcoRI site of pBR 322. Plasmid pOCT3 was kindly provided by Professor K. Matsubara at Osaka University, School of Medicine. The trp operon DNA is cut out from pOCT3 by digestion with EcoRI and, once isolated, it is then put back to the EcoRI site of pOCT3 in such a manner that the cut ends become switched around, leading to pOCT2. Thus the transcriptional direction of pOCT3 is opposite to that of pOCT2; it is clockwise in pOCT2 (5' to 3') whereas counterclockwise in pOCT3. Fig. 4 shows the DNA sequence (about 300 bases) in the vicinity of the trp promoter in pOCT2 plasmid.

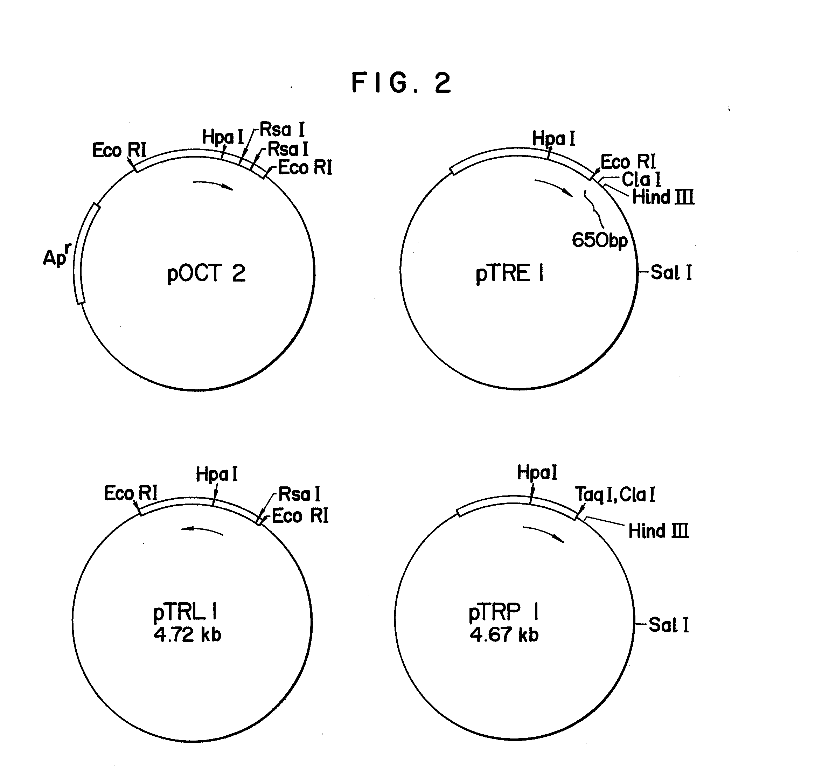

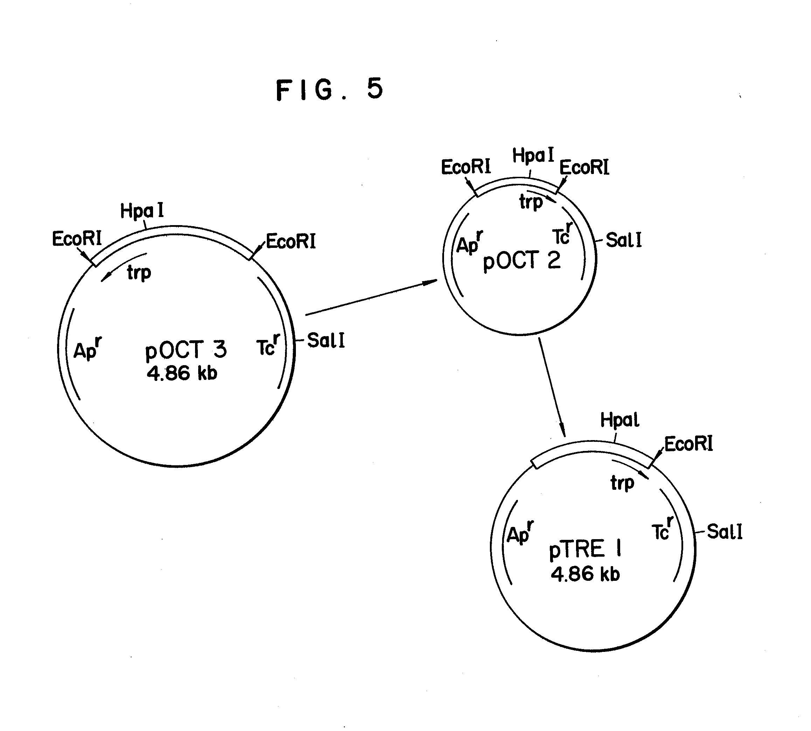

Fig. 5 is a schematic diagram for the construction of pOCT3 from pOCT2. A restriction enzyme map for pOCT2 is shown in FIG. 2.

pTRE1

Plasmid pTRE1 is derived from pOCT2 and has a single EcoRI site since the other EcoRI site near the ampicillin resistant region has been removed from pOCT2. This plasmid contains the intact trp operon part of pOCT2 as shown in FIG. 2. The construction of pTRE1 is accomplished in the following manner.

Plasmid pOCT2 is partially digested with EcoRI, resulting in the cleavage of the EcoRI site near the ampicillin resistant region. The single stranded-portions are then repaired by means of DNA polymerase and the repaired ends are joined using $T_4$ DNA ligase to produce pTRE1.

A restriction enzyme map for pTRE1 is shown in FIG. 2. Fig. 5 shows a schematic diagram for the construction of pTRE1 from pOCT2.

pTRL1

Plasmid pTRL1 has a DNA portion from pOCT2 bounded by EcoRI and RSaI sites. This portion comprises 360 base pairs (from an unidentified EcoR site to RSaI site in the trp L region), some of which are sequenced as shown in FIG. 4. The construction of pTRL1 is accomplished in the following manner.

Plasmid pOCT2 is cleaved with EcoRI and then partially digested with RSaI to yield a 360 base pair fragment containing the trp promoter region (trp L). This fragment is ligated with the EcoRI linker at the cut RSaI site. Digestion with EcoRI produces a fragment with cohesive ends. This fragment is then inserted into the EcoRI site of pBR322 to afford two kinds of recombinant plasmids. Plasmid pTRL1 is the one wherein the transcriptional direction from the trp promoter is counter-clockwise.

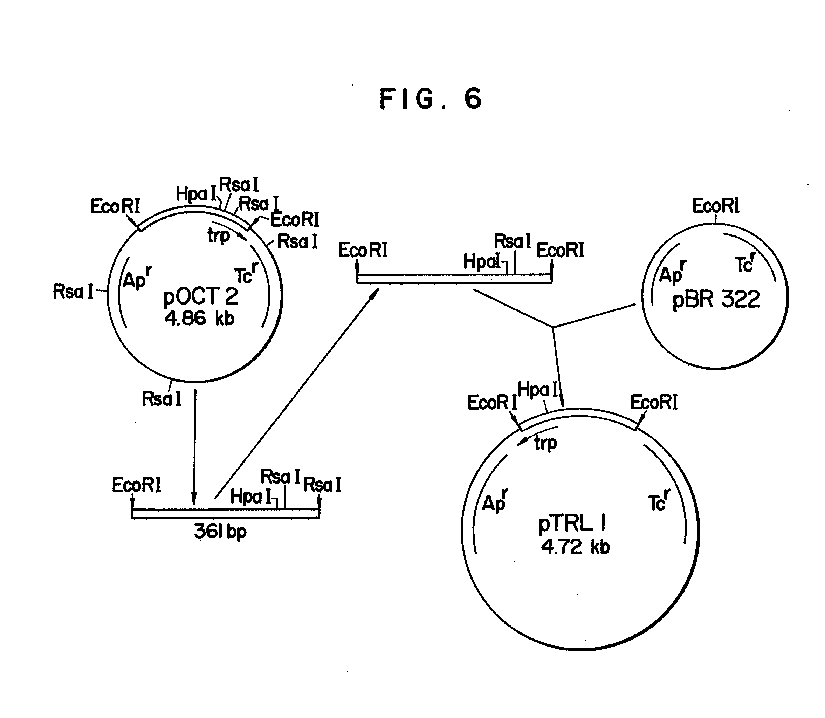

A restriction enzyme map for pTRL1 is shown in FIG. 2. FIG. 6 is a schematic diagram for the construction of pTRL1 from pOCT2.

pTRP1

Plasmid pTRP1 has a DNA portion from pTRE1 bounded by HpaI and ClaI sites. This portion comprises about 4640 base pairs originally derived from pBR322. The plasmid has also another DNA portion from pTRE1 bounded by HpaI and TaqI sites. This portion comprises 32 base pairs corresponding to the trp L promoter. The

construction of pTRP1 is accomplished in the following manner.

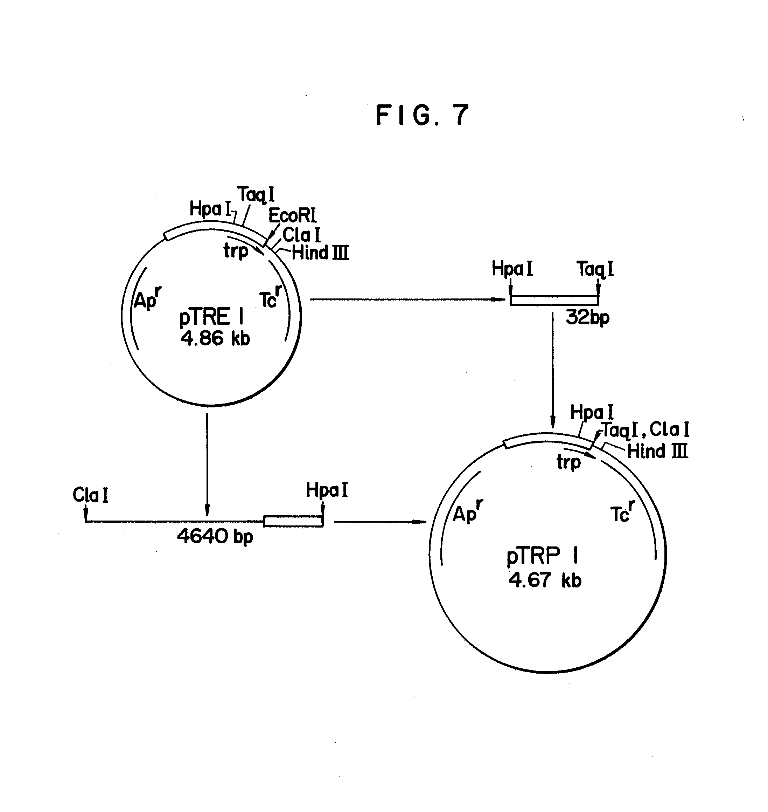

Plasmid pTRE1 is cleaved with HpaI and ClaI to yield an about 4640 bp linear DNA fragment. Both fragments are joined together by ligation to produce pTRP1. A restriction enzyme map for pTRP1 is shown in FIG. 2. FIG. 7 is a schematic diagram for the construction of pTRP1 from pTRE1.

Expression Plasmids

Again using standard techniques as outlined previously, a desired DNA fragment containing the cloned prochymosin gene is extracted from a transformant, and cleaved with restriction enzymes; the vector plasmid is similarly cleaved; and the fragments are mixed and ligated. These steps have led to the construction of expression plasmids, pCR501, pCR601, and pCR701 which are designed to facilitate to obtain expression of prochymosin in E. coli.

pCR501

Plasmid pCR501 contains a DNA portion of pTRE1 fused to about 1,000 nucleotides from pCR301, which code for the procymosin molecule. The construction of pCR501 is accomplished in the following manner.

Plasmid pTRE1 is cut with EcoRI and single-stranded portions are removed by means of S1 nuclease. The EcoRI linker (GGATTTCC/CCTTAAGG) is linked to the blunt end using $T_4$ DNA ligase. This DNA is next digested with EcoRI and SalI to produce linear DNA segment (a) (ca. 4210bp). Plasmid pCR301 is cut with EcoRI and KpnI to obtain DNA segment (b). This DNA segment is a 246 bp sequence containing the prochymosin gene fused to the residual beta-glactosidase coding gene. Similarly, pCR301 is cut with KpnI and SalI to obtain DNA segment (c). This DNA is a 1025 bp sequence of the prochymosin gene including its C-terminus. Each DNA segment is ligated together by means of $T_4$ DNA ligase. E. coli strain C600 is transformed with the ligated DNA, and ampicillin resistant colonies (pCR501) are selected.

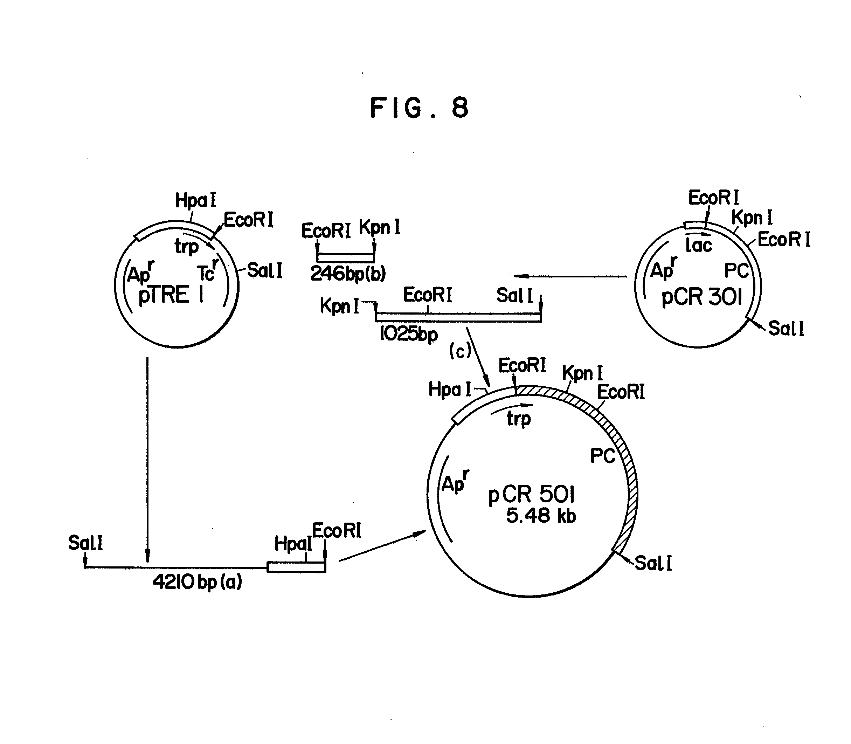

As shown in FIG. 3, analysis of pCR501 by restriction enzyme clevage has revealed that pCR501 contains the trp operon promoter and about 1,000 nucleotides coding for prochymosin (nucleotide #13 to the end of the gene, viz. 5th codon to 365th). FIG. 8 shows a schematic diagram for the construction of pCR501 from pCR301 and pTRE1.

Following the method of Maxam-Gilbert, the nucleotide sequence between the initiation ATG codon and the beginning of prochymosin gene (5th codon) has been analyzed to be:

```
                                          EcoRI
                                            ▽
  5' - ATG CAA ACA CAA AAA CCG ACT GGG GAA TTC CGG - 3'
        Met                                    Arg

           trp E region                 prochymosin
```

Transformed cells carrying the plasmid will express the cloned gene and produce prochymosin under the control of the trp E promoter.

The prochymosin produced in this manner lacks an amino acid sequence of the genuine prochymosin which corresponds to that from the 1st to the 4th amino acid at the N terminus, but contains, in its place, eight amino acids (methionine and others) resulting from the above-stated trp E promoter coding sequence as well as two amino acid residues resulting from beta-glactosidase. This prochymosin is therefore called prochymosin like protein.

pCR601

Plasmid pCR601 contains a DNA portion of pTRE1 and a 56 bp fragment from pTRL1 fused to the same coding sequence for prochymosin present in pCR501. The construction of pCR601 is accomplished in the following manner.

Plasmid pTRE1 is cut with HpaI and SalI to produce a linear 4010 bp segment (DNA (d)). Next, plasmid pTRL1 is cut with HpaI and EcoRI to produce a 56 bp segment (DNA (e)) containing the trp L promoter. both segments are mixed and ligated together with DNA (b) and (c) (both obtained previously from pCR301), by means of $T_4$ DNA ligase. E. coli strain c 600 is transformed with the resulting ligated DNA and ampicillin resistant colonies (pCR 601) are selected.

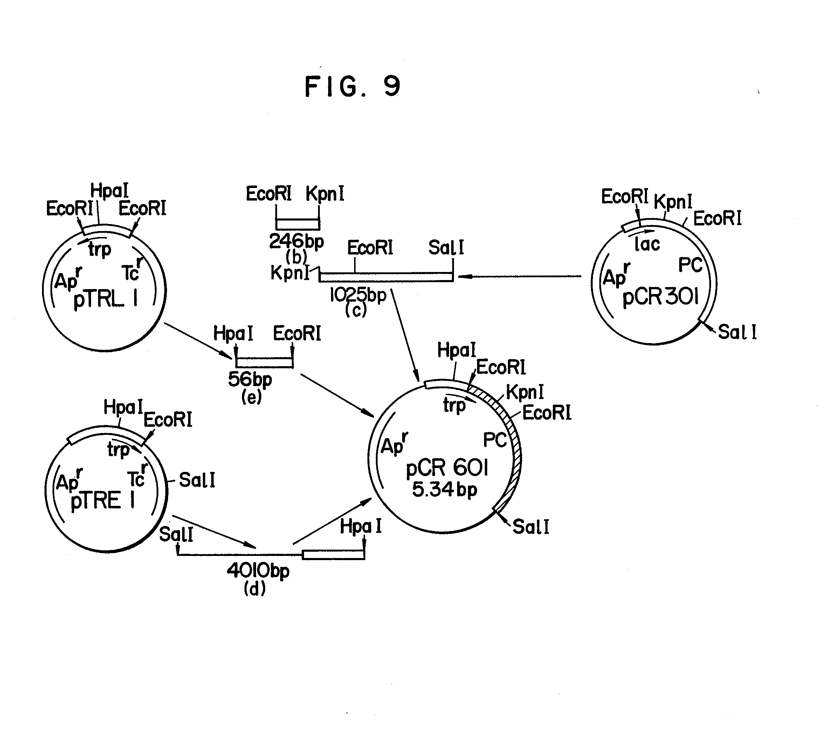

As shown in FIG. 3, analysis of pCR601 by restriction enzyme cleavage has revealed that pCR601 contains the trp L promoter and about 1,000 nucleotides coding for prochymosin (nucleotide #13 to the end of the gene, viz, 5th codon to 365th). FIG. 9 shows a schematic diagram for the construction of pCR601 from pCR301, pTRE1 and pTRL1.

Following the method of Maxam-Gilbert, the nucleotide sequence between the initiation ATG codon and the beginning of prochymosn gene (5th codon) has been analyzed to be:

```
                            EcoRI
                              ▽
 5' - ATG AAA GCA ATT TTC GTG GAA TTC CGG - 3'
      Met                                 Arg  prochymosin

             trp L region
```

Transformed cells carrying the plasmid will express the cloned prochymosin gene and produce prochymosin under the control of trp L promoter.

The prochymosin produced in this manner lacks an amino acid sequence of the genuine prochymosin which corresponds to that from the 1st to the 4th amino acid at the N terminus, but contains, in its place, six amino acids (methionine and others) resulting from the above-stated trp L promoter coding sequence as well as two amino acid residues resulting from beta-glactosidase. This prochymosin is therefore called prochymosin like protein.

<u>pCR701</u>

Plasmid pCR701 contains a DNA portion of pTRPI and a synthetic nucleotide piece fused to a prochymosin coding sequence from pCR501. The construction of pCR701 is accomplished in the following manner.

Plasmid pTRP1 is cut with Hind III and Sal I to obtain liner DNA segment (f) (about 4050 bp). Plasmid pCR501 is cut with BamHI and SalI to obtain liner DNA segment (g) (1264 bp). A synthetic oligonucleotide having a 22bp is synthesized, which is the sequence of:

5' AGCTTATGGCTGAGATCACCAG 3'

3' ATACCGACTCTAGTGGTCCTAG 5'

This DNA segment (f) makes up for the nucleotide sequence that has been removed by digestion with Bam HI which cuts the 14th nucleotide of prochymosin gene. The three segments (f), (g) and (h) are ligated together by means of $T_4$ DNA ligase. <u>E</u>. <u>coli</u> c600 is transformed with the ligated DNA and ampicillin resistant colonies (pCR701) are selected.

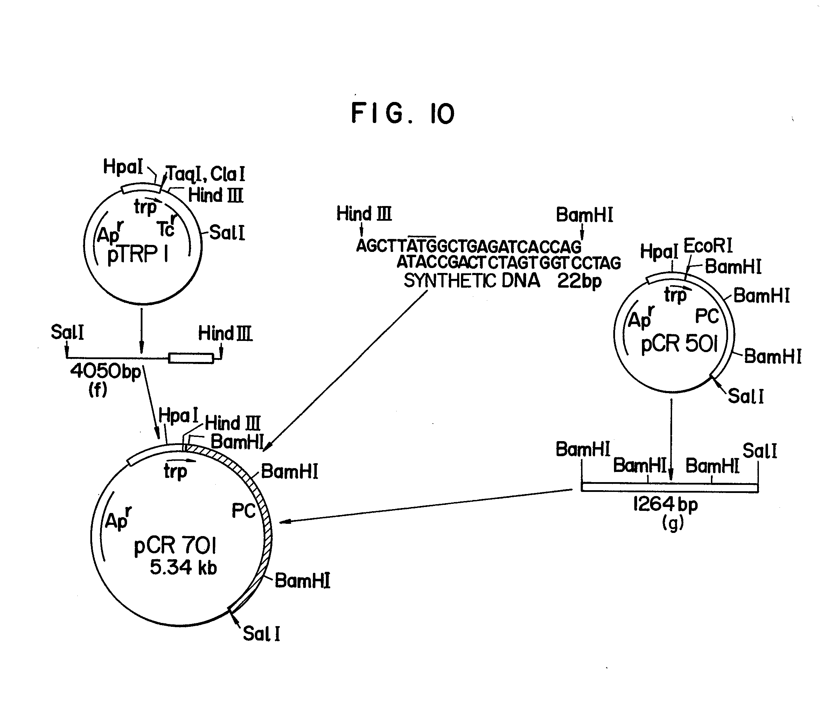

As shown in FIG. 3, analysis of pCR701 by restriction enzyme cleavage has revealed that pCR701 contains the <u>trp</u> L promoter and the entire prochymosin coding sequence (i.e. nucleotide #1 to the end of the gene, <u>v12</u>, 1st codon to 365th). FIG. 10 shows a schematic diagram for the construction of pCR701 from pTRP1 and pCR501.

As determined by the method of maxam-Gilbert, the SD (Shinon and Delgarno) region is located 13 nucleotides away from the ATG initiation codon preceeding the lst codon of the prochymosin gene (see below).

```
                    TaqI  HindIII
        5'
         _  AAGG GTA TCGATAAGCTT ATG GCT - 3'
            SD                   Met Ala
                    ClaI
```

Transformed cells carrying the plasmid will express the prochymosin gene and produce prochymosin under the control of the trp L promoter.

The prochymosin produced in this manner is N-terminal methionylprochymosin in which methionine arising from the initiation codon ATG is fused to the N terminus of the genuine prochymosin.

Expression of prochymosin in E. coli

A portion of the full-growth culture (in LB medium) of E. coli cells transformed with the expression plasmids is grown in M9 broth containing 3-beta-indolylacrylic acid (15 µg/ml) and ampicillin (50 µg/ml). Cells are harvested when propagation reaches maximum. The cells are then destroyed by sonication treatment and a cell-free extract is obtained. This extract is subjected to electrophoresis in a polyacrylamide gel containing SDS. The product protein is detected by the protein blotting method. Following the above procedures or radioimmunoassay, each transformant strain containing pCR501, pCR601 or pCR701 has been tested for its ability to produce prochymosin. Analysis by autoradiography (Nishimori, Gene, 19, 337 (1982) has revealed that every strain gives a protein band the size of which is equal to that of the authentic prochymosin. In addition, the level of production of prochymosin has been estimated to be more than 300,000 molecules per cell. Similar results have been obtained by an analysis using radioimmunoassay.

These results have shown that prochymosin is produced in E. coli cells carrying plasmid pCR501, pCR601 or pCR701 under the transcriptional control of the E. coli trp operon promoter and that the levels of production are substantially greater than those previously reported under the control of the E. coli lac operon. Even higher levels of production will be possible by using the same scheme in accordance with this invention and by obtaining fusions of the trp promoter closer to the initiation codon of prochymosin which preferably proceeds the tip of the prochymosin gene.

E. coli strains prepared by processes according to this invention are exemplified by cultures deposited with the Fermentation Research Institute, Ibaraki, Japan under the Budapest Treaty, and are identified by the Accession number designated to each strain.

This invention will be described in some detail by way of illustration and example; nevertheless, it should be understood that certain changes and modifications be practiced within the scope of the invention.

EXAMPLE

Throughout the following examples, TEN buffer solution is meant to be a solution containing Tris-HCl(20mM), NaCl(50mM) and EDTA(1mM) with a pH of about 7.5.

Example 1.

Preparation of pTRE1 Plasmid

Plasmid pOCT2 was partially digested with EcoRI by incubation at 20°C for 7.5 min in a 30 µl solution containing Plasmid (1.5 µg in 15 µl TEN), EcoRI (2.5 U, 0.5 µl TEN), EcoRI buffer (3 µl), and $H_2O$ (11.5 µl). After argarose gel electrophoresis, the desired DNA was isolated (in 40 µl TEN) and this was heated at 60°C for 5 min. To this DNA solution (40 µl) was added DNA polymerase I (4 U, 4 µl TEN), 16 mM ATP (4 µl), polymerase buffer solution (6 µl), and $H_2O$ (2 µl). The resulting mixture (a total volume of 60 µl) was incubated at 25°C for 30 min. After addition of 0.25 M EDTA solution (4 µl) followed by phenol and ether treatment, DNA was precipitated with ethanol. The precipitate was dissolved in a solution (10 µl) containing $T_4$ DNA ligase (1 U, 1 µl), 3 mM ATP (2 µl), ligase buffer solution (2 µl) and $H_2O$ (5 µl), and this was incubated at 22°C for 2 h. After incubation, the DNA was precipitated with ethanol and dissolved in TEN solution. This solution was used to transform the calcium-treated E. coli C 600 strain. One of twelve colonies, there was obtained two transformants containing pTRE1.

Example 2

## Preparation of pTRL1 Plasmid

i) Preparation of DNA Segment (1)

Plasmid pOCT2 was cut with EcoRI at 37° C for 2 h in a 120 μl solution containing Plasmid (10.6 μg in 100 μl of TEN), EcoRI buffer (12 μl), and EcoRI (40 U, 8 μl of TEN). After incubation, the DNA was precipitated with ethanol and the precipitate was dissolved in 50 μl of TEN. The cut DNA was partially digested with RsaI by incubation at 20°C for 10 min in a 60 μl solution containing DNA (49 μl of TEN), BamHI buffer (6 μl), $H_2O$ (3 μl), and RsaI (20 U, 2 μl of TEN). The doubly cut DNA was precipitated with ethanol and the precipitate was dissolved in 20 μl of TEN. The dissolved solution was then applied to polyacrylamide gel electrophoresis. After electrophoresis, a 364 bp linear DNA (0.16 μg in 40 μl of TEN) was isolated. Meanwhile, EcoRI linker was kinased with $T_4$ polynucleotide kinase at 37°C for 1 h in a 20 μl reaction mixture containing the linker (5 μg, 5 μl TEN), ATP (3mM, 2 μl), kination buffer (2 μl), $H_2O$ (9 μl), and the kinase (22U in 2 μl TEN). The kination mixture (20 μl) was added to the above DNA solution (35

μl), followed by addition of $T_4$ DNA ligase (5U, 5 μl TEN), ligase buffer (4 μl), and ATP (6mM, 7 μl). The total mixture (71 μl) was ligated together by incubation at 22°C for 2 h. The ligated DNA was precipitated with ethanol and the precipitate was dissolved in 50 μl TEN. To the dissolved solution (45 μl) was added EcoRI (350 U in 70 μl TEN), EcoRI buffer (20 μl) and $H_2O$ (65 μl). The resulting mixture (200 μl) was incubated at 37°C for 3 h. Incubation was terminated by phenol treatment and DNA was extracted with ether, precipitated with ethanol, and dissolved in 40 μl of TEN. The dissolved solution was applied to polyacrylamide gel electrophoresis. After electrophoresis, the DNA segment (1) with cohesive ends (0.1 μg in 240 μl of Tris-acetate buffer) was isolated.

ii) Preparation of DNA Segment (2) from pBR322

Plasmid pBR322 was cut with EcoRI by incubation at 37°C for 1 h in a 30 μl solution containing plasmid (4.5 μg in 20 μl TEN), EcoRI buffer (3 μl), $H_2O$ (3 μl), and EcoRI (20 U in 4 μl TEN). Incubation was terminated by phenol treatment and DNA was extracted with ether, precipitated with ethanol, and dissolved in 40 μl of 10 mM Tris-HCl buffer. The dissolved solution was treated with 10 μl of MATE BAP by incubation at 65°C fr 1 h. After incubation, MATE BAT was removed by washing the incubated mixture with 20 μl of 10mM Tris-HCl buffer. Thus the DNA segment (2) (4.2 μg) was

recovered in 60 μl of 10 mM Tris-HCl buffer.

iii) Ligation of DNA Segment (1) and (2)

Segment (1) (0.05 μg in 120 μl of Tris-acetate buffer) and Segment (2) (0.14 μg in 2 μl of 10 mM Tris-HCl buffer) were mixed and the mixed solution was treated with ethanol.  The resulting precipitate was dissolved in 50 μl of TEN.  Forty microliters of the dissolved solution was subjected to ligation using $T_4$ DNA ligase (0.9 U in 1 μl TEN), ligase buffer (5 μl) and ATP (6 mM, 5 μl TEN) by incubation a 51 μl solution at 22°C for 2 h.  The ligated DNA was precipitated with ethanol and dissolved in 50 μl of TEN.

iv) Transformation

Twenty microliters of the above ligated DNA solution was used to transform competent cells of strain E. coli C 600.  About one thousand five hundred transformants were found to be amplicillin resistant. Restriction enzyme analysis demonstrated that one out of twenty transformants contained pTRL1 plasmid.

Preparation of pTR1 Plasmid

i) Preparation of DNA Segment (3) from pTREI Plasmid

Plasmid pTRE1 was cut with HpaI by incubation at 37°C for 1.5 h in a 40 μl solution containing Plasmid (2.46 μg in 30 μl TEN), HpaI buffer (4 μl), and HpaI (30 U in 6 μl TEN).  The cut DNA was further cut with ClaI by adding the DNA solution to a 10 μl solution con-

taining ClaI buffer (5 µl) and ClaI (25 U, 5 µl TEN), and by incubating at 37°C for 1.5 h. The doubly cut DNA was precipitated with ethanol and the precipitate was dissolved in 40 µl of TEN. The dissolved solution was applied to agarose gel electrophoresis. After electrophoresis, the 4640 bp linear DNA Segment (3) (2.35 µg in 80 µl TEN) was isolated.

ii) Preparation of DNA Segment (4) from pTRE1 Plasmid

Plasmid pTRE1 was cut with HpaI at 37°C for 1.5 h in a 120 µl solution containing Plasmid (8.2 µg in 100 µl TEN), HpaI buffer (12 µl), and HpaI (57 U in 8 µl TEN). To the above mixture was added a solution containing TaqI (80 U in 9 µl TEN) and HpaI buffer (12 µl), and HpaI (57 U in 8 µl TEN). To the above mixture was added a solution containing TaqI (80 U in 9 µl TEN) and HpaI buffer (1 µl). The resulting mixture (130 µl) was incubated at 65°C for 1 h. After incubation, the doubly cut DNA was precipitated with ethanol and the precipitate was dissolved in 40 µl TEN. The dissolved solution was applied to polyacrylamide gel electrophoresis. After electrophoresis, the 32 bp linear DNA segment (4) (0.054 µg in 40 µl TEN) was isolated.

iii) Ligation of DNA Segment (3) and (4)

The mixture containing DNA Segment (3) (0.29 µg in 10 µl TEN), Segment (4) (0.014 µg in 10 µl TEN) was subjected to ligation using $T_4$ ligase (3.6 U in 4 µl

- 28 -

0121775

TEN), ligase buffer (3 μl), and ATP (6 mM, 3 μl TEN). The ligation mixture (30 μl) was incubated at 22°C for 2 h. The ligated DNA was precipitated with ethanol and the precipitate was dissolved in 40 μl TEN.

iv) Transformation

Thirty microliters of the above TEN solution containing the ligated DNA was used to transform competent cells of strain E. coli C600. About eight hundred and forty transformants were found to be ampicillin resistant. All the transformants contained pTRP1 plasmid.

Example 4

Construction of pCR501 Plasmid

i) Preparation of DNA Segment (a) from pTRE1 Plasmid

Plasmid pTRE1 was cleaved by incubation at 37°C for 2 h in a 120 μl reaction mixture containing Plasmid (8.2 μg in 100 μl TEN), EcoRI buffer (12 μl), $H_2O$ (3 μl), and EcoRI (25 U in 5 μl TEN). After phenol extraction, ether extraction, ethanol precipitation, and dissolution in 50 μl TEN, the cut DNA was further cut with S1 nuclease at 22°C for ½ h in a 50 μl reaction mixture containing DNA (25 μl TEN), SI buffer (5 μl), $H_2O$ (10 μl), and SI nuclease (100 U, 10 μl). Incubation was terminated by phenol treatment and DNA was extracted with ether and precipitated with ethanol. In the meantime, EcoRI linker was kinased with $T_4$ poly-

nucleotide kinase at 37°C for 1 h in a 20 µl reaction mixture containing the linker (5 µg, 5 µl), ATP (3mM, 2 µl), kination buffer (2 µl), $H_2O$ (9 µl) and Kinase (22 U in 2 µl TEN). The kination mixture (20 µl) containing EcoRI linker was added to the ethanol precipitated cut DNA along with ATP (3 mM, 5 µl) plus 4.5 units of $T_4$ ligase (5 µlTEN). The mixture (30 µl) was incubated at 22°C for 2 h. The product DNA was precipitated with ethanol and the precipitate was dissolved in 60 µl TEN. To the DNA solution (50 µl) was added EcoRI (500 U in 50 µl TEN), EcoRI buffer (20 µl), and $H_2O$ (80 µl). The resulting mixture (200 µl) was incubated at 37°C for 2 h. Incubation was terminated by phenol treatment and DNA was extracted with ether and precipitated with ethanol. The precipitated DNA was cut with SalI by incubation at 37°C for 1.5 h in a 110 µl solution containing SalI (100 U, 20 µl), SalI buffer (10 µl), $H_2O$ (80 µl). After incubation, the cut DNA was precipitated with ethanol and dissolved in 60 µl TEN. The DNA was subjected to agarose gel electrophoresis, yielding 3.5 µg of the purified linear DNA (a)(4210 bp) in 100 µl TEN.

ii) Preparation of DNA Segment (b) from pCR301 Plasmid

Plasmid pCR301 was cut with KpnI by incubation at 37°C for 1.5 h in a 120 µl solution containing Plasmid (7.3 µg in 100 µl TEN), KpnI buffer (12 µl),

$H_2O$ (2 μl), and KpnI (35 U, 6 μl TEN). To the mixture was added EcoRI buffer solution (14 μl) containing EcoRI (30 U, 6 μl TEN). The 140 μl mixture was incubated at 37°C for 1 h. The doubly cut DNA was precipitated with ethanol and the precipitate was dissolved in 60 μl TEN. The dissolved solution was applied to polyacrylamide gel electrophoresis. After electrophoresis, the 246 bp DNA Segment (b) (0.03 μg in 4 μl TEN) was isolated.

iii) Preparation of DNA Segment (c) from pCR301 Plasmid

Plasmid pCR301 was cut with KpnI by incubation at 37°C for 1.5 h in a 120 μl solution containing Plasmid (15 μg in 100 μl TEN), KpnI buffer (12 μl), $H_2O$ (2 μl), and KpnI (35 U, 6 μl TEN). A 14 μl SalI buffer solution plus SalI (32 U, 4 μl TEN), $H_2O$ (2 μl) was added to the above incubated mixture. The 140 μl mixture was further incubated at 37°C for 1 h. The doubly cut DNA was precipitated with ethanol and the precipitate was dissolved in 60 μl TEN. The dissolved solution was applied to agarose gel electrophoresis. After electrophoresis, the 1025 bp DNA Segment (c) (2.15 μg in 180 μl TEN) was isolated.

iv) Ligation of DNA Segments (a), (b) and (c)

The mixture containing DNA Segment (a) (1 μg in 30 μl TEN), Segment (b) (0.03 μg in 4 μl TEN), and Segment (c) (0.4 μg in 30 μl TEN) was lypophilized. After lypophilization, the precipitate was rinsed with ethanol. Each precipitated segment was ligated together in a 20 μl solution containing ligase buffer (4 μl), ATP (3 mM, 4 μl), $H_2O$ (1 μl) and $T_4$ ligase (0.9 U, 1 μl). Incubation was continued for 2 h at 22°C. The ligated DNA was precipitated with thanol and dissolved in 40 μl TEN.

v) Transformation

The TEN buffer solution (30 μl) containing the above ligated DNA was employed for transformation of E. coli strain C600. Twenty transformants were found to contain plasmid pCR501. One of them was deposited with the FRI as E. coli C600 $r_k^- m_k^-$ (pCR501) FERM BP 262.

Example 5

Construction of pCR601 Plasmid

i) Preparation of DNA Segment (d) from pTRE1 Plasmid

Plasmid pTRE1 was cut with HpaI by incubation at 37°C for 1 h in a 40 μl solution containing Plasmid (2.46 μg in 30 μl TEN), HpaI buffer (4 μl), and HpaI (35 U, 6 μl TEN). To the incubated mixture was then added a 10 μl solution containing SalI buffer (5 μl) and SalI (20 U, 5 μl TEN). The mixture (50 μl) was further incubated at 37°C for 1 h. The doubly cut DNA was precipitated with ethanol and the precipitate was dissolved in 40 μl TEN. The dissolved solution was applied to agarose gel electrophoresis. After electrophoresis, the 4010 bp Segment (d) (2.0 μg in 40 μl TEN) was isolated.

ii) Preparation of DNA Segment (e) from pTRL1
Plasmid

Plasmid pTRL1 was cut with HpaI by incubation at 37°C for 1.5 h in a 120 μl solution containing Plasmid (15.4 μg in 100 μl TEN), HpaI buffer (12 μl), and HpaI (25 U, 5 μl TEN), and $H_2O$ (3 μl). To the incubated mixture was then added a 20 μl solution containing EcoRI buffer (914 μl) and EcoRI (30 U, 6 μl TEN). The mixture (140 μl) was further incubated at 37°C for 1 h. The doubly cut DNA was precipitated with ethanol and the precipitate was dissolved in 40 μl TEN. The dissolved solution was applied to polyacrylamide gel electrophoresis. After electrophoresis, the 56 bp Segment (e) (0.18 μg in 40 μl TEN) was isolated.

iii) Preparation of DNA Segment (b) from
pCR301 Plasmid

By repeating the procedure of Example 4, but using 32 units of KpnI and 30 units of EcoRI, plasmid pCR301 was cut with KpnI and then with EcoRI to afford 0.46 μg (in 40 μl TEN) of the 246 bp DNA segment after polyacrylamide gel electrophoresis.

iv) Preparation of DNA Segment (c) from pCR301
Plasmid

By repeating the procedure of Example 4, but using 30 units of KpnI and 24 units of SalI, plasmid pCR301 was cut with KpnI and then with SalI to afford 3.9 μg (in 40 μl TEN) of the 1025 bp DNA segment after agarose gel electrohporesis.

v) Ligation of DNA Segments (b), (c), (d) and (e)

The mixture containing DNA Segment (b) (0.06 μg in 5 μl TEN) Segment 9c) (0.49 μg in 5 μl TEN), Segment (d) (0.5 μg in 10 μl TEN), and Segment (e) (0.49 μg in 5 μl TEN) was subjected to ligation using $T_4$ ligase (5.5 U in 6 μl TEN) and ligation buffer (4.5 μl) plus ATP (6 mM, 4.5 μl). The ligated mixture (45 μl) was incubated at 22°C for 2 h. The ligated DNA was precipitated with ethanol and dissolved in 60 μl TEN.

vi) Transformation

The TEN buffer solution ( 20 μl) containing the above ligated DNA was used to transform E. coli C600 $r_k^- m_k^-$ strain. Twenty seven ampicillin resistant transformants were obtained after transformation. Eight transformants were found to contain pCR601 as determined by restriction enzyme digestion. This strain E. coli C600 $r_k^- m_k^-$ (pCR601) was deposited with the FRI as FERM BP263.

Example 6

Construction of pCR701 Plasmid

i) Preparation of DNA Segment (f) from pTRP 1 Plasmid

Plasmid pTRP1 was cut with HindIII by incubation at 37°C for 1 h in a 120 μl solution containing Plasmid (25.2 ˉg in 100 μl TEN), Hind III buffer (12

μl), and Hind III (48 U, 8 μl TEN). To the incubated mixture was added SalI (24U, 6 μl TEN) and SalI buffer (14 μl). The resulting 140 μl solution was incubated at 37°C for 2 h. The doubly cut DNA was precipitated with ethanol and the precipitate was dissolved in 60 μl TEN. The dissolved solution was applied to agarose gel electrophoresis. After electrophoresis, the 4051 bp linear DNA Segment (f) (8.2 μg in 120 μl TEN) was isolated.

ii) Preparation of DNA Segment (g) from pCR501 Plasmid

Plasmid pCR501 was cut with SalI by incubation at 37°C for 1 h in a 120 μl solution containing Plasmid (39 μg in 100 μl TEN), SalI buffer (12 μl), and SalI (32 U, 8 μl TEN). The cut DNA was partially digested with BamH I by addition of BamH I (25 U, 5 μl TEN) to the above mixture and by incubation at 22°C for ½ h. The product DNA was precipitated with ethanol and the precipitate was dissolved in 60 μl TEN. This dissolved solution was applied to agarose gel electrophoresis. After electrophoresis, the 1264 bp DNA Segment (g) (2 μg in 80 μl TEN) was isolated.

iii) DNA Segment (h)

A synthetic oligonucleoide having the following sequence was obtained:

```
5'    AGCTTATGGCTGAGATCACCAG    3'
         ATACCGACTCTAGTGGTCCTAG    5'
```

iv) Ligation of DNA Segments (f), (g) and (h)

The mixture containing DNA Segment (f) (0.68 µg in 10 µl TEN), Segment (g) (0.25 µg in 10 µl TEN), and Segment (h) (0.046 µg in 10 µl TEN) was subjected to ligation using $T_4$ DNA ligase (1.8 U, in 2 µl TEN) and ligation buffer (4 µl) plus ATP (6 mM, 4 µl TEN). The ligated mixture DNA was precipitated with ethanol and dissolved in 50 µl TEN.

v) Transformation

Twenty microliters of the above ligation reaction mixture was used to transform competent cells of E. coli C600. Eight ampicillin resistant transformants were obtained and, by restriction enzyme analysis, four of these transformants were found to have pCR701. This strain E. coli C600 $r_k-m_k-$ (pCR701) was deposited with the FRI as FERM BP 264.

Example 7

Expression of Prochymosin in E. coli

E. coli strain harboring pCR501, pCR601 or pCR701 was preliminarily cultivated in LB at 37°C overnight. One mililiter of the culture broth was then transferred to M9 medium (10 ml), and cultivated for 1 h. Cultivation was continued at 37°C for additional 3 to 4 h aftr 3-beta-indolylacrylic acid (15 mg/l) was added to the medium. Cells were harvested by centrifugation at 15,000 rmp for about 15 min, and suspended in 3 ml of PBS buffer (150 mM NaCl in 20 mM sodium

0121775

phosphate buffer, pH 7.0) which contains 1 mM PMSF (phenylmethylsulfonyl fluoride). To this suspention was added 60 µl of 250 mM EDTA and 30 µl lysozyme (10 ml/l) and the mixture was kept at 0°C for ½ h. The resulting sheroplasts were disrupted by sonication and to this was added 3.09 ml of 8 M urea. The cell lysate mixture was incubated at 37°C for 1 h. After centrifugation at 3000 rmp for ½ h, the supernatant solution was dialyzed against PBS. The dialysate was applied to the binding competitive radioimmunoassay (see, Nishimori et al., Gene). By radioimmunoassay prochymosin was immunoprecipitated and the amount of the radioactive elemnt ($^{125}$I) was measured with a gamma-ray counter. In the meantime, calibration curves were prepared by varying the concentration of authentic prochymosin. By comparing the count of the immunoprecipitated prochymosin with the calibration curves, the amount of prochymosin in the cell lysate was determined. This experiment demonstrated that the expression level of prochymosin was highest with the culture broth from E. coli containing pCR501 (about 300,000 molecules per host cell) and lowest with that from E. coli containing pCR701.

Independantly, the above sonicated cell-free extract was treated with 4M urea. After centrifugation, the supernantant solution was applied to SDS-polyacrylamide gel electrophoresis, and the migrated proteins were then blotted onto nitrocellulose filters.

These filters were analyzed on autoradiography as previously described in Nishimori et al., Gene. For each bacterial extract, a distinctive band corresponding to that of prochymosin was observed. By comparing the density of these bands, it was determined that E. coli containing pCR501 plasmid produced about 300,000 prochymosin molecules per cell. The ability of each expression plasmid-containing host cell to produce prochymosin was evaluated to be in the order of pCR501 $\geq$ PCR601 > pCR701. This trend is well in accord with the results from the radioimmunoassay method.

<u>Renaturation and Activation of Bacterial Prochymosin and Milk-Clotting Activity</u>

Total sonicates of plasmid-containing bacteria were treated with 8M urea and dialyzed against 25 mM sodium bicarbonate (pH 10.0) in order to remove urea. After another dialysis against 50 mM Tris-HCl buffer (pH 7.5), dialysates were activated by addition of 0.4 M glycin (pH 2.3) and by maintaining the solution at room temperature for 15 min.

The milk-clotting activity of each bacterial extract was tested according to the method of Foltman (B. Foltman, Prochymosin and Chymosin Methods in Enzymology, <u>19</u>, 42 (1972). In this test, the milk-clotting activity to coagulate 10 ml of skim milk within 100 sec at 30° may be defined as one chymosin unit (CU). For each bacterial extract derived from the expression plasmids, the milk-clotting activity was measured after activation of the prochymosin so-produced (v_iz_. conversion of bacterial prochymosin to chymosin); the activity for pCR501 was 9.3 CU/mg and 3.7 CU/mg for pCR701. It should be recognized that a bacterial extract harboring no expression plasmid displayed no milk-clotting activity at all as determined by the above testing procedures.

- 39 -

WHAT IS CLAIMED IS:

1.      A method for expressing a prochymosin coding gene in E. coli host cells, wherein said gene is derived from a recombinant plasmid containing cloned prochymosin cDNA, said method comprising:

isolating said gene; attaching by ligation a transcriptional promoter and a translational initiation codon thereto to form an expression plasmid; introducing the expression plasmid into host cells by transformation; and selecting transformed cells that have high levels of expression of prochymosin, said gene having at least from the fifth coding codon to the end of the coding sequence (365th codon) for prochymosin.

2.      A method according to claim 1 wherein said recombinant plasmid is plasmid pCR 301; said host cells are E. coli c 600 $r^-_k m^-_k$; and said transcriptional promoter is the E. coli trp operon promoter.

3.      A method according to claim 1 wherein said expression plasmid is pCR501, pCR601 or pCR701.

4.      A method for expressing a prochymosin coding gene in E. coli host cells, wherein said gene is derived from a recombinant plasmid containing cloned prochymosin cDNA, said method comprising:

isolating said gene; ligating onto said gene a synthetic oligonucleotide carrying a complementary portion of the prochymosin coding sequence which has been deleted from said gene; attaching thereto by ligation a transcriptional promotor and a translational initiation codon when not present in said synthetic oligonucleotide to form an expression plasmid; introducing the expression plasmid into host cells by transformation; and selecting transformed cells that have high levels of expression of prochymosin, said synthetic oligonucleotide optionally carrying a translational initation codon.

5.      A method according to claim 4 wherein said recombinant plasmid is pCR301 plasmid; said host cells are E. coli c 600 $r^-_k m^-_k$; and said transcriptional promoter is the E. coli trp operon promoter.

6.      A method according to claim 4 wherein said expression plasmid is pCR701.

7.      An expression plasmid comprising a prochymosin coding gene and a vector operatively attached thereto, said vector being originally derived from pBR322 plasmid and containing a transcriptional promoter and a translational initiation codon.

8.      An expression plasmid according to claim 7 wherein said prochymosin coding gene is the nucleotide sequence from the fifth codon to the end of the coding sequence (365th codon); said transcriptional promoter is the E. coli trp operon promoter; and said translational initiation codon is ATG codon.

9.      An expression plasmid according to claim 7 wherein said prochymosin coding gene is a nucleotide sequence from the first codon to the end of the coding sequence (365th codon) for prochymosin; and said transcriptional promoter is the E. coli trp operon promoter.

10. An expression plasmid according to claim 8 wherein said translational initiation codon is within the trp L or trp E region.

11. An expression plasmid according to claim 10 wherein said coding gene is fused to the following DNA sequence at its 5' end

$$5'\text{-}\underline{AGAG}AATTAC\underline{AATG}CAAACACAAAAACCGACTGGGGAATTC\text{-}3'$$
$$\phantom{5'\text{-}}SD$$

12. An expression plasmid according to claim 10 wherein said coding gene is fusd to the following DNA sequence at its 5' end

$$5'\text{-}\underline{AAGGG}TATCGAC\underline{AATG}AAAGCAATTTTCGTGGAATTC\text{-}3'$$
$$\phantom{5'\text{-}}SD$$

13. An expression plasmid according to claim 9 wherein said translational initiation codon is within the trp L region.

14. An expression plasmid according to claim 13 wherein said translational initiation codon proceeds the first codon of the prorennin coding sequence.

15. An expression plasmid according to claim 14 wherein said coding gene is fused to the following DNA sequence at its 5' end:

$$5'\text{-}\underline{AAGGG}TATCGATAAGCT\underline{ATG}\text{-}3'$$
$$\phantom{5'\text{-}}SD$$

0121775

- 42 -

16.     Prochymosin gene comprising a nucleotide
sequence from the fist codon (GCT) to the 365th (ATC)
codon.

17.     Prochymosin gene according to claim 16 which
further comprising ATG codon proceeding said first
codon.

18.     Prochymosin gene comprising a nucleotide
sequence from the fifth codon (CGG) to the 365th codn
(ATC).

19.     A method for making pTRE1 plasmid which
comprises the steps of:

    (1)   partially digesting pOCT2 with EcoRI in order
        to cut the EcoRI site closer to the $Ap^r$ gene
        portion;

    (2)   repairing the cut single-stranded DNA parts by
        means of DNA polymerase; and

    (3)   combining the repaired cut ends together by
        ligation using $T_4$ DNA ligase.

20.     A method for making pTRL 1 plasmid which
comprises the steps of:

    (a)   digesting pOCT2 with EcoRI and partially with
        RsaI to produce a linear DNA segment;

    (b)   ligating thereto the kinased EcoRI linker
        followed by digestion with EcoRI to produce
        DNA segment (1);

    (c)   digesting pBR322 plasmid with EcoRI followed
        by treatment with MATE BAP to produce DNA segment (2); and

(d)   ligating DNA segments (1) and (2) by means of

T$_4$ DNA ligase.

21.   A method for making pTRP1 plasmid which comprises the steps of:

(a)   digesting pTRE1 plasmid with HpaI and ClaI successively to produce DNA segment (3);

(b)   digesting pTRE1 with HpaI and TaqI successively to produce DNA segment (4); and

(c)   ligating DNA segments (3) and (4) by means of T$_4$ DNA ligase.

22.   A method for making pCR501 plasmid which comprises the steps of:

(a)   digesting pTRE 1 plasmid with EcoRI and S1 successively to produce a linear DNA segment with blunt ends;

(b)   ligating the EcoRI linker thereto followed by digestion with EcoRI and SalI to produce linear DNA segment (a);

(c)   digesting pCR 301 with KpnI and EcoRI successively to produce linear DNA Segment (b);

(d)   independently digesting pCR301 with KpnI plus SalI to produce DNA segment (c); and

(e)   ligating DNA segments (a), (b) and (c) together by means of T$_4$ DNA ligase.

23.   A method for making pCR601 plasmid which comprises the steps of:

(a) digesting pTRE1 plasmid with HpaI and SalI successively to produce linear DNA segment (d);

(b) digesting pTRL1 plasmid with HpaI and EcoRI successively to produce linear DNA segment (e);

(c) digesting pCR301 plasmid with KpnI plus EcoRI, and with KpnI plus Sal I respectively to produce DNA segment (b) and segment (c); and

(d) ligating DNA segments (b), (c), (d) and (e) together by means of $T_4$ DNA ligase.

24. A method for making pCR701 plasmid which comprises the steps of:

(a) digesting pTRP1 plasmid with HindIII and SalI successively to produce linear DNA segment (f);

(b) digesting pCR501 plasmid with SalI and partially with BamHI to produce DNA segment (g);

(c) obtaining a synthetic oligonucleotide (DNA segment (h)) having the nucleotide sequence of

```
                       3'
5' AGCTTATGGCTGAGATCACCAG
       ATACCGACTCTAGTGGTCCTAG5'
     3'                           ; and
```

(d) ligating DNA segments (f), (g) and (h) together by means of $T_4$ DNA ligase.

25. A recombinant plasmid found in E. coli FRI Accession number FERM BP 262.

26.    A Recombinant plasmid found in E. coli FRI
Accession number FERM BP 263.

27.    A Recombinant plasmid found in E. coli FRI
Accession number FERM BP 264.

28.    A method for producing prochymosin by means of
expression of a prochymosin coding gene in E. coli host
cells,

wherein said gene is derived from a recombinant plasmid containing cloned prochymosin cDNA, said
method comprising:

isolating said gene; attaching by ligation a
transcriptional promoter and a translational initiation
codon thereto to form an expression plasmid; introducing
the expression plasmid into host cells by
transformation; selecting transformed cells that have
high levels of expression of prochymosin; and growing
said cells in a nutrient medium.

29.    A method according to claim 28 wherein said
recombinant plasmid is pCR301 plasmid; said host cells
are derived from E. coli C 600 $r_k^- m_k^-$; and said
transcriptional promoter is the E. coli trp operon
promoter.

30.    A method according to claim 28 wherein said
expression plasmid is selected from the group consisting
of pCR501, pCR601, and pCR701; and said host cells are
derived from E. coli C 600 $r_k^- m_k^-$.

31.    A method according to claim 28 wherein said transformed cells are found in a deposited E. coli strain selected from the group consisting of Accession number FERM BP262, FERM BP263 and FERM BP264.

32.      Calf Prochymosin produced by the method of claim 28.

33.      E. coli strain capable of producing prochymosin which contains an expression plasmid, said plasmid being selected from the group consisting of pCR501, pCR601 and pCR701.

34.      E. coli strain as deposited in the FRI Accession number FERM BP262.

35.      E. coli strain as deposited in the FRI Accession number FERM BP263.

36.      E. coli strain as deposited in the FRI Accession number FERM BP264.

# FIG. la

```
                                                                    -20
                                                    TGCTACTTGCTGTCTTCGCTCTCTCCCAGGGC
                                                    LeuLeuAlaValPheAlaLeuSerGlnGly

            20                 40                 60                 80                100
GCTGAGATCACCAGGATCCCTCTGTACAAAGGCAAGTCTCTGAGGAAGGCGCTGAAGGAGCATGGGCTTCTGGAGGACTTCCTGCAGAAACAGCAGTATG
AlaGluIleThrArgIleProLeuTyrLysGlyLysSerLeuArgLysAlaLeuLysGluHisGlyLeuLeuGluAspPheLeuGlnLysGlnGlnTyrG
                    10                                        20                                 30

            120                140                160                180                200
GCATCAGCAGCAAGTACTCCGGCTTCGGGGAGGTGGCCAGCGTGCCCCTGACCAACTACCTGGATAGTCAGTACTTTGGGAAGATCTACCTCGGGACCCC
lyIleSerSerLysTyrSerGlyPheGlyGluValAlaSerValProLeuThrAsnTyrLeuAspSerGlnTyrPheGlyLysIleTyrLeuGlyThrPr
                    40                                        50                                 60

            220                240                260                280                300
GCCCCAGGAGTTCACCGTGCTGTTTGACACTGGCTCCTCTGACTTCTGGGTACCCTCTATCTACTGCAAGAGCAATGCCTGCAAAAACCACCAGCGCTTC
oProGlnGluPheThrValLeuPheAspThrGlySerSerAspPheTrpValProSerIleTyrCysLysSerAsnAlaCysLysAsnHisGlnArgPhe
                    70                                        80                                 90                      100

            320                340                360                380                400
GACCCGAGAAAGTCGTCCACCTTCCAGAACCTGGGCAAGCCCCTGTCTATCCACTACGGGACAGGCAGCATGCAGGGCATCCTGGGCTATGACACCGTCA
AspProArgLysSerSerThrPheGlnAsnLeuGlyLysProLeuSerIleHisTyrGlyThrGlySerMetGlnGlyIleLeuGlyTyrAspThrValT
                    110                                       120                                130

            420                440                460                480                500
CTGTCTCCAACATTGTGGACATCCAGCAGACAGTAGGCCTGAGCACCCAGGAGCCCGGGGACGTCTTCACCTATGCCGAATTCGACGGGATCCTGGGGAT
hrValSerAsnIleValAspIleGlnGlnThrValGlyLeuSerThrGlnGluProGlyAspValPheThrTyrAlaGluPheAspGlyIleLeuGlyMe
                    140                                       150                                160

            520                540                560                580                600
GGCCTACCCCTCGCTCGCCTCAGAGTACTCGATACCCGTGTTTGACAACATGATGAACAGGCACCTGGTGGCCCAAGACCTGTTCTCGGTTTACATGGAC
tAlaTyrProSerLeuAlaSerGluTyrSerIleProValPheAspAsnMetMetAsnArgHisLeuValAlaGlnAspLeuPheSerValTyrMetAsp
                    170                                       180                                190                     200
```

# FIG. 1b

<pre>
         620              640              660              680              700
AGGAATGGCCAGGAGAGCATGCTCACGCTGGGGGCCATCGACCCGTCCTACTACACAGGGTCCCTGCACTGGGTGCCCGTGACAGTGCAGCAGTACTGGC
ArgAsnGlyGlnGluSerMetLeuThrLeuGlyAlaIleAspProSerTyrTyrThrGlySerLeuHisTrpValProValThrValGlnGlnTyrTrpG
(Asp)              210                           220                           230

         720              740              760              780              800
AGTTCACTGTGGACAGTGTCACCATCAGCGGTGTGGTTGTGGCCTGTGAGGGTGGCTGTCAGGCCATCCTGGACACGGGCACCTCCAAGCTGGTCGGGCC
lnPheThrValAspSerValThrIleSerGlyValValValAlaCysGluGlyGlyCysGlnAlaIleLeuAspThrGlyThrSerLysLeuValGlyPr
                   240                           250                           260

         820              840              860              880              900
CAGCAGCGACATCCTCAACATCCAGCAGGCCATTGGAGCCACACAGAACCAGTACGATGAGTTTGACATCGACTGCGACAACCTGAGCTACATGCCCACT
oSerSerAspIleLeuAsnIleGlnGlnAlaIleGlyAlaThrGlnAsnGlnTyrAspGluPheAspIleAspCysAspAsnLeuSerTyrMetProThr
                270                           280                           290                           300

         920              940              960              980              1000
GTGGTCTTTGAGATCAATGGCAAAATGTACCCACTGACCCCCTCCGCCTATACCAGCCAAGACCAGGGCTTCTGTACCAGTGGCTTCCAGAGTGAAAATC
ValValPheGluIleAsnGlyLysMetTyrProLeuThrProSerAlaTyrThrSerGlnAspGlnGlyPheCysThrSerGlyPheGlnSerGluAsnH
                   310                           320                           330

         1020             1040             1060             1080             1100
ATTCCCAGAAATGGATCCTGGGGGATGTTTTCATCCGAGAGTATTACAGCGTCTTTGACAGGGCCAACAACCTCGTGGGGCTGGCCAAAACCATCTGATC
isSerGlnLysTrpIleLeuGlyAspValPheIleArgGluTyrTyrSerValPheAspArgAlaAsnAsnLeuValGlyLeuAlaLysThrIle *
                   340                           350                           360       (Ala)

         1120             1140             1160             1180             1200
ACATCGCTGACCAAGAACCTCACTGTCCCCACACACCTGCACACACACATGCACACATGTACATGAGCACATGTGCACACACACAGATGAGGTTTCCAGA
        *

         1220             1240
CAGATGATTCTCAATAAACGTTGTCTTTCTGCAAAAAAAAAAAAAAAA
        ———————
</pre>

0121775

FIG. 2

FIG. 3

pCR 501
trp operon HpaI EcoRI BamHI KpnI
PROCHYMOSIN
SalI

pCR 701
trp operon HindⅢ BamHI
PROCHYMOSIN
SalI

pCR 301
Ap^r lac PO BamHI KpnI BamHI
PROCHYMOSIN
BamHI SalI

pCR 601
trp operon EcoRI EcoRI KpnI
PROCHYMOSIN
SalI

## FIG. 4

<sup>5'</sup>CTCAAGGCGCACTCCCGTTCTGGATAATGTTTTTTGCGCCGA

CATCATAACGGTTCTGGCAAATATTCTGAAATGAGCTGTTGA

                   HpaI      RsaI

CAATTAATCATCGAACTAGTTAACTAGTACGCAAGTTCACGT

           TaqI                            RsaI

TrpL  AAAAAGGGTATCGACA ATG AAAGCAATTTTCGTACTGAAAGG
                SD

TTGGTGGCGCACTTCCTGAAACGGGCAGTGTATTCACCATGC

GTAAAGCAATCAGATACCCAGCCCGCCTAATGAGCGGGCTTT

TrpE  TTTTTGAACAAAATTAGAGAATAACA ATG CAAACACAAAAAC
          EcoRI         SD

CGACTGGAATTCTC<sup>3'</sup>

FIG. 5

FIG. 6

# FIG. 7

FIG. 8

FIG. 9

FIG. 10

| | DOCUMENTS CONSIDERED TO BE RELEVANT | | EP 84102451.6 |

| Category | Citation of document with indication, where appropriate, of relevant passages | Relevant to claim | CLASSIFICATION OF THE APPLICATION (Int. Cl. ³) |
|---|---|---|---|
| X,D | <u>EP - A2 - 0 073 029</u> (BEPPU)<br><br>   * Abstract; claims 1-4,10-13, 17-21 *<br><br>& JP-A2-56-131 631, Kokai-no. 32896/83<br><br>-- | 1,2,4, 5,7, 28-30, 32 | C 12 N 15/00<br>C 12 N 9/50//<br>C 12 R 1/19 |
| A,D | GENE, vol. 19, 1982, (Amsterdam, NL)<br><br>K. NISHIMORI et al.: "Expression of cloned calf prochymosin gene sequence in Escherichia coli" pages 337-344<br><br>   * The whole article *<br><br>-- | 1,2,4, 5,7-9, 28,29 | |
| A,D | THE JOURNAL OF BIOCHEMISTRY, vol. 90, 1981, (Tokyo, JP)<br><br>K. NISHIMORI et al.: "Cloning in Escherichia coli of the Structural Gene of Prorennin, the Precursor of Calf Milk-Clotting Enzyme Rennin" pages 901-904<br><br>   * The whole article *<br><br>-- | 1,2,4, 5,7-9, 28,29 | TECHNICAL FIELDS SEARCHED (Int. Cl. ³)<br><br>C 12 N |
| A,D | <u>EP - A2 - 0 068 691</u> (CELLTECH LIMITED)<br><br>   * Abstract; claim 4 *<br><br>-- | 1,4,28 | |
| A,D | <u>EP - A2 - 0 057 350</u> (COLLABORA-TIVE RESEARCH INC.)<br><br>   * Claims 7-9 *<br><br>---- | 1,4,28 | |

The present search report has been drawn up for all claims

| Place of search | Date of completion of the search | Examiner |
|---|---|---|
| VIENNA | 07-06-1984 | WOLF |